# EUROPÄISCHE PATENTSCHRIFT

(11) **EP 1 077 672 B1**
(45) Veröffentlichungstag und Bekanntmachung des Hinweises auf die Patenterteilung: **05.06.2002**
(21) Anmeldenummer: 99917931.0
(22) Anmeldetag: 31.03.1999
(51) Int. Cl.: A61K 7/42, A61K 7/48

(54) **SONNENSCHUTZMITTEL**
SUNSCREENS
AGENTS DE PROTECTION SOLAIRE

(30) Priorität: 06.04.1998 DE 19815086
(43) Veröffentlichungstag der Anmeldung: 28.02.2001
(73) Patentinhaber: Cognis Deutschland GmbH & Co. KG, 40589 Düsseldorf (DE)
(72) Erfinder: HÖRNER, Viola, D-40593 Düsseldorf (DE); KÜHNE, Sabine, D-42781 Haan (DE); WACHTER, Rolf, D-40595 Düsseldorf (DE)
(86) Internationale Anmeldenummer: EP9902203
(87) Internationale Veröffentlichungsnummer: WO9951197

(56) Entgegenhaltungen:
- WO-A-95/01773
- WO-A-98/38966
- DE-A- 4 323 615
- FR-A- 2 511 243
- US-A- 4 707 354

## Beschreibung

### Gebiet der Erfindung

Die Erfindung betrifft kosmetische Mittel enthaltend Desoxyribonucleinsäuren und UV-Lichtschutzfilter, sowie deren Verwendung zur Herstellung von Sonnenschutzmittel und After-Sun Präparaten.

### Stand der Technik

Unter dem Einfluß von Sonnenstrahlung kommt es zur Pigmentierung normaler Haut durch die Bildung von Melaninen. Dabei ruft die Bestrahlung mit langwelligem UV-A Licht die Dunkelung der in der Epidermis bereits vorhandenen Melaninkörper hervor, ohne daß schädigende Folgen zu erkennen sind, während die kurzwellige UV-B Strahlung die Bildung neuen Melanins bewirkt. Ehe das schützende Pigment jedoch gebildet werden kann, unterliegt die Haut der Einwirkung der ungefilterten Strahlung, die je nach Expositionsdauer zu Hautrötungen (Erythemen), Hautentzündungen (Sonnenbrand) oder gar Brandblasen führen kann. Die mit derartigen Hautläsionen verbundenen Belastungen des Organismus, beispielsweise im Zusammenhang mit der Ausschüttung von Histaminen, kann zusätzlich zu Kopfschmerzen, Mattigkeit, Fieber, Herz- und Kreislaufstörungen und dergleichen führen. Daneben können Langzeitexpositionen zu kumulativer DNA-Schädigung führen, die in Hautkrebs resultieren kann. Für den Verbraucher, der sich vor den schädlichen Aspekten der Sonneneinstrahlung schützen will, bietet der Markt eine Vielzahl von Produkten, bei denen es sich ganz überwiegend um Öle und milchige Emulsionen handelt, die neben einigen Pflegestoffen vor allem synthetische UV-Lichtschutzfilter enthalten. So beschreibt die **US 4,707,354** (Alpen Tau Inc.) kosmetische Mittel mit einer Kombination von UV-Lichtschutzfiltem und Allantoin. Übersichten zu synthetischen UV-Lichtschutzfiltem finden sich beispielsweise in P. Finkel in **Parf.Kosm. 76, 432 (1995)** und S. Schauder in **Parf.Kosm. 76, 490 (1995).**

Dennoch besteht im Markt weiterhin das Bedürfnis nach Produkten mit einem verbesserten Leistungsspektrum. Für die Leistungsbeurteilung solcher Produkte ist vor allem relevant, inwiefern sie in der Lage sind, die schädigenden Wirkungen der UV-Strahlung auf die Haut und insbesondere auf die Nucleinsäuren der Hautzellen zu verhindern. Des weiteren sind Hautverträglichkeit sowie der Einsatz natürlicher Produkte beim Kunden gefragt.

Besonderes Interesse gilt dabei Zubereitungen, die eine Einarbeitung von größeren Mengen UV-Lichtschutzfiltern erlauben, ohne däß im Laufe der Lagerung eine Phasentrennung bzw. eine Sedimentation stattfindet. Eine nach der Phaseninversionstemperaturmethode hergestellte Formulierung, wie beispielsweise in der Europäischen Patentanmeldung **EP-A1 066 144** (L'Oreal) beschrieben, neigt bei der Einarbeitung von größeren Mengen Titandioxid sehr rasch zur Ausscheidung des dispergierten Feststoffes. Ein weiteres Problem besteht darin, daß viele UV-Lichtschutzfilter mit den weiteren Bestandteilen der Rezeptur in Wechselwirkung treten können, was zu einer chemischen Reaktion und ebenfalls zu einer Abnahme der Lagerbeständigkeit führt. Schließlich wünscht der Verbraucher Formulierungen, die auch gegenüber sehr empfindlicher Haut eine hohe hautkosmetische Verträglichkeit aufweisen, wie z.B. Gele. Die komplexe Aufgabe der Erfindung hat somit darin bestanden, Sonnenschutzmittel zur Verfügung zu stellen, die sich gleichzeitig durch besondere Phasenstabilität, Lagerbeständigkeit sowie Transparenz und Verträglichkeit gegenüber empfindlicher Haut auszeichnen.

### Beschreibung der Erfindung

Gegenstand der Erfindung sind kosmetische Mittel, enthaltend
(a) Desoxyribonucleinsäuren und
(b) UV-Lichtschulzfilter.

Überraschenderweise wurde gefunden, daß durch die Kombination von Desoxyribonucleinsäuren und UV-Lichtschutzfiltern Produkte erhalten werden, die ein Absorptionsspektrum zeigen, das sich für den Einsatz in Sonnenschutzmitteln eignet und gleichzeitig eine hohe Hautverträglichkeit aufweist. Durch den Einsatz von Desoxyribonudeinsäuren weisen diese Mittel ein Absorptionsspektrum auf, welches zum Schutz der körpereigenen Nucleinsäuren besonders geeignet ist. Da die eingesetzten Desoxyribonucleinsäuren ein identisches Absorptionsspektrum wie die körpereigenen Nucleinsäuren besitzen, ist nicht nur eine sehr effektive UV-Absorption gewährleistet, sondern es wird gleichzeitig noch ein antioxidativer Schutz erreicht: die schädigenden Wirkungen der UV-Strahlen erreichen zunächst die Desoxyribonucleinsäuren der kosmetischen Mittel und schützen so die körpereigenen Nucleinsäuren vor strahleninduzierten Schäden. Die Kombination von Desoxyribonucleinsäuren mit UV-Lichtschutzfiltern führt somit zu einem besonders effektiven Schutz der Haut vor den schädlichen Wirkungen der Sonnenstrahlung bei gleichzeitig hoher Verträglichkeit dieser Mittel. Diese Wirkung ist alleine durch die Kombination bekannter Lichtschutzfaktoren nicht möglich. Des weiteren ist es möglich durch geeignete Wahl der Komponente (b) Mittel zu erhalten, die ausschließlich auf Basis natürlicher Rohstoffe hergestellt werden. Dies ist besonders hinsichtlich der Verträglichkeit der Endprodukte von Bedeutung. In einer Ausführungsform der Erfindung liegen die Komponenten (a) und (b) in Form von sog. Sonnenschutzgelen auf Basis wässriger oder wässrig-alkoholischer Lösungen vor. In einer weiteren Ausführungsform können diese Komponenten in Form von Sonnenölen eingesetzt werden.

Ebenfalls Gegenstand der Erfindung ist, daß als weitere Komponenten (c) Emulgatoren und (d) Ölkörper eingesetzt werden können. Je nach Wahl der Komponenten (c) und (d) kann man O/W oder W/O Emulsionen enthalten (Sonnenmilch und Sonnencremes). Die erfindungsgemäßen Sonnenschutzmittel zeichnen sich durch Phasenstabilität und Lagerbeständigkeit aus. Durch die Kombination von UV-Lichtschutzfiltern mit Desoxyribonucleinsäuren ist es möglich, stabile Produkte mit besonders hohem Schutzfaktor für die Haut zu erhalten, die gleichzeitig eine gute Hautverträglichkeit zeigen.

### Desoxyribonucleinsäuren

Unter Desoxyribonucleinsäuren (DNA) werden hochmolekulare, fadenförmige Polynucleotide verstanden, die sich von 2'-Desoxy-β-D-ribonucleosiden ableiten, die ihrerseits wieder von äquivalenten Mengen einer Nucleobase und der Pentose 2-Desoxy-D-ribo-furanose aufgebaut werden. Der Einsatz von Nucleinsäuren als Wirkstoffe in der Kosmetik ist bekannt. So werden beispielsweise in der französischen Patentanmeldung **FR-A1 2511253** Haut- und Sonnenschutzmittel mit einem Gehalt an hochpolymerisierter DNA vorgeschlagen. Aus der japanischen Offenlegungsschrift **JP-A2 62/096404** (Kanebo) sind kosmetische Zusammensetzungen mit Nucleinsäuren und Diisopropylamindichloracetat bekannt. Gegenstand der französischen Patentschrift **FR-B1 2620024** (Soc.d'Etudes Dermatologiques) sind Zubereitungen, enthaltend Nucleinsäurederivate als Radikalfänger. Beispiele sind Adenin, Guanosin, Xanthin, Hypoxanthin, Uracil und Ribonucleinsäure. In der internationalen Patentanmeldung WO **95/01773** (Boston University) wird ein Verfahren zur Stimulation der Pigmentproduktion beschrieben, bei dem man DNA-Fragmente, bevorzugt Dinucleotide, in liposomaler Form in die Epidermis transportiert. Gegenstand der deutschen Patentanmeldung **DE-A1 4323615** sind schließlich Zusammensetzungen mit einem Gehalt an Nucleinsäuren und deren Fragmenten als Anti-Ageing- und Sonnenschutzcremes. Aus der **DE-T1 3990820** (Laboratoires Serobiologique) sind weiterhin Lichtschutzmittel auf Basis von Ribonucleinsäuren bekannt, ebenso wie aus dem US-Patent **US 5,039,516** (Dento-Med Industries).

FR-A-2 511 243 offenbart Kosmetische Zusammensetzungen mit höheren DNA gehalten. Die DNA erfillt eine Nähr funktion.

Als Nucleobasen kann die DNA die Purinderivate Adenin und Guanin sowie die Pyrimidine Cytosin und Thymin enthalten. In den Nucleinsäuren sind die Nucleobasen N-glykosidisch mit Kohlenstoffatom 1 der Ribose verknüpft, wodurch im Einzelfall Adenosine, Guanosine, Cytidine und Thymidine entstehen. In den Säuren verknüpft eine Phosphatgruppe die 5'-Hydroxygruppe der Nucleoside mit der 3'-OH-Gruppe der jeweils folgenden durch eine Phosphodiesterbrücke unter Ausbildung von Einzelstrang-DNA. In der Regel liegt die DNA doppelsträngig vor (Ausbildung von Wasserstoffbrückenbindungen zwischen den entsprechenden Basen). Je nach Behandlung der Nucleinsäuren, kann die DNA sowohl in Form von Doppelsträngen und/oder Einzelsträngen vorliegen. Unter dem Begriff Desoxyribonucleinsäuren im Sinne der vorliegenden Erfindung werden sowohl doppel- als auch einzelsträngige Desoxyribonucleinsäuren verstanden, des weiteren Mischungen aus Einzelsträngen und Doppelsträngen. Wegen des großen Verhältnisses von Länge zu Durchmesser neigen DNA-Moleküle schon bei mechanischer Beanspruchung, etwa bei der Extraktion, zu Strangbruch. Aus diesem Grunde kann das Molekulargewicht der Nucleinsäuren 10³ bis 10⁹ Dalton erreichen. Im Sinne der Erfindung werden konzentrierte DNA-Lösungen eingesetzt, die sich durch ein flüssig-kristallines Verhalten auszeichnen. Vorzugsweise werden Desoxyribonucleinsäuren eingesetzt, die aus marinen Quellen beispielsweise durch Extraktion von Fischsperma erhalten werden und die ein Molekulargewicht im Bereich von 1000 bis 5.000.000 Dalton aufweisen. Besonders bevorzugt ist der Einsatz von einzelsträngigen Desoxyribonucleinsäuren marinen Ursprungs mit einem Molekulargewicht im Bereich von 10.000 bis 100.000 Dalton. Die Desoxyribonucleinsäuren können in den erfindungsgemäßen Mitteln in Mengen von 0,001 bis 2,5, vorzugsweise 0,1 bis 0,5 Gew.-% - bezogen auf das Mittel - enthalten sein.

### UV-Lichtschutzfilter

Unter **UV-Lichtschutfiltern** sind organische Substanzen zu verstehen, die in der Lage sind, ultraviolette Strahlen zu absorbieren und die aufgenommene Energie in Form längerwelliger Strahlung, z.B. Wärme wieder abzugeben. UVB-Filter können öllöslich oder wasserlöslich sein. Als öllösliche Substanzen sind z.B. zu nennen:
- 3-Benzylidencampher und dessen Derivate, z.B. 3-(4-Methylbenzyliden)campher;
- 4-Aminobenzoesäurederivate, vorzugsweise 4-(Dimethylamino)benzoesäure-2-ethylhexylester, 4-(Dimethylamino)benzoesäure-2-octylester und 4-(Dimethylamino)benzoesäureamylester;
- Ester der Zimtsäure, vorzugsweise 4-Methoxyzimtsäure-2-ethylhexylester, 4-Methoxyzimtsäurepropylester, 4-Methoxyzimtsäureisoamylester, 2-Cyano-3-phenyl-zimtsäure-2-ethylhexylester (Octocrylene);
- Ester der Salicylsäure, vorzugsweise Salicylsäure-2-ethylhexylester, Salicylsäure-4-isopropylbenzylester, Salicylsäurehomomenthylester;
- Derivate des Benzophenons, vorzugsweise 2-Hydroxy-4-methoxybenzophenon, 2-Hydroxy-4-methoxy-4'-methylbenzophenon, 2,2'-Dihydroxy-4-methoxybenzophenon;
- Ester der Benzalmalonsäure, vorzugsweise 4-Methoxybenzmalonsäuredi-2-ethylhexylester;
- Triazinderivate, wie z.B. 2,4,6-Trianilino-(p-carbo-2'-ethyl-1'-hexyloxy)-1,3,5-triazin und Octyltriazon.
- Propan-1,3-dione, wie z.B. 1-(4-tert.Butylphenyl)-3-(4'methoxyphenyl)propan-1,3-dion.

Als wasserlösliche Substanzen kommen in Frage:
- 2-Phenylbenzimidazol-5-sulfonsäure und deren Alkali-, Erdalkali-, Ammonium-, Alkylammonium-, Alkanolammonium- und Glucammoniumsalze;
- Sulfonsäurederivate von Benzophenonen, vorzugsweise 2-Hydroxy-4-methoxybenzophenon-5-sulfonsäure und ihre Salze;
- Sulfonsäurederivate des 3-Benzylidencamphers, wie z.B. 4-(2-Oxo-3-bomylidenmethyl)benzolsulfonsäure und 2-Methyl-5-(2-oxo-3-bornyliden)sulfonsäure und deren Salze.

Besonders bevorzugt ist die Verwendung von Estern der Zimtsäure, vorzugsweise 4-Methoxyzimtsäure-2-ethylhexylester, 4-Methoxyzimtsäureiso-pentylester, 2-Cyano-3-phenyl-zimtsäure-2-ethylhexylester (Octocrylene). Desweiteren ist die Verwendung von Derivaten des Benzophenons, insbesondere 2-Hydroxy-4-methoxybenzophenon, 2-Hydroxy-4-methoxy-4'-methylbenzophenon, 2,2'-Dihydroxy-4-methoxybenzophenon sowie der Einsatz von Propan-1,3-dionen, wie z.B. 1-(4-tert.Butylphenyl)-3-(4'methoxyphenyl)propan-1,3-dion bevorzugt.

Als typische UV-A-Filter kommen insbesondere Derivate des Benzoylmethans in Frage, wie beispielsweise 1-(4'-tert.Butylphenyl)-3-(4'-methoxyphenyl)propan-1,3-dion oder 1-Phenyl-3-(4'-isopropylphenyl)-propan-1,3-dion. Die UV-A und UV-B-Filter können selbstverständlich auch in Mischungen eingesetzt werden. Neben den genannten löslichen Stoffen kommen für diesen Zweck auch unlösliche Pigmente, nämlich feindisperse Metalloxide bzw. Salze in Frage, wie beispielsweise Titandioxid, Zinkoxid, Eisenoxid, Aluminiumoxid, Ceroxid, Zirkoniumoxid, Silicate (Talk), Bariumsulfat und Zinkstearat. Die Partikel sollten dabei einen mittleren Durchmesser von weniger als 100 nm, vorzugsweise zwischen 5 und 50 nm und insbesondere zwischen 15 und 30 nm aufweisen. Sie können eine sphärische Form aufweisen, es können jedoch auch solche Partikel zum Einsatz kommen, die eine ellipsoide oder in sonstiger. Weise von der sphärischen Gestalt abweichende Form besitzen. Neben den beiden vorgenannten Gruppen primärer Lichtschutzstoffe können auch sekundäre Lichtschutzmittel vom Typ der Antioxidantien eingesetzt werden, die die photochemische Reaktionskette unterbrechen, welche ausgelöst wird, wenn UV-Strahlung in die Haut eindringt. Typische Beispiele hierfür sind Superoxid-Dismutase, Tocopherole (Vitamin E) und Ascorbinsäure (Vitamin C). Weitere geeignete UV-Lichtschutzfilter sind der Übersicht von P.Finkel in **SÖFW-Journal 122, 543 (1996)** zu entnehmen. Der Anteil der Lichtschutzmittel an den efindungsgemäßen Mitteln liegt - bezogen auf die Mittelüblicherweise bei 1 bis 20, vorzugsweise 5 bis 15 Gew.%. Die erfindungsgemäßen Mittel als solche können 1 bis 95, vorzugsweise 5 bis 80 und insbesondere 10 bis 60 Gew.-% Wasser enthalten. Werden als Lichtschutzmittel organische Verbindungen eingesetzt, können zur Herstellung der Zubereitungen deren co-emulgierenden Eigenschaften mitgenutzt werden.

### Emulgatoren

Als **Emulgatoren** (Komponente c) kommen beispielsweise nichtionogene Tenside aus mindestens einer der folgenden Gruppen in Frage:
(c1) Anlagerungsprodukte von 2 bis 30 Mol Ethylenoxid und/ oder 0 bis 5 Mol Propylenoxid an lineare Fettalkohole mit 8 bis 22 C-Atomen, an Fettsäuren mit 12 bis 22 C-Atomen und an Alkylphenole mit 8 bis 15 C-Atomen in der Alkylgruppe;
(c2) C_{12/18}-Fettsäuremono- und -diester von Anlagerungsprodukten von 1 bis 30 Mol Ethylenoxid an Glycerin;
(c3) Glycerinmono- und -diester und Sorbitanmono- und -diester von gesättigten und ungesättigten Fettsäuren mit 6 bis 22 Kohlenstoffatomen und deren Ethylenoxidanlagerungsprodukte;
(c4) Alkylmono- und -oligoglycoside mit 8 bis 22 Kohlenstoffatomen im Alkylrest und deren ethoxylierte Analoga;
(c5) Anlagerungsprodukte von 15 bis 60 Mol Ethylenoxid an Ricinusöl und/oder gehärtetes Ricinusöl;
(c6) Polyol- und insbesondere Polyglycerinester, wie z.B. Polyglycerinpolyricinoleat, Polyglycerinpoly-12-hydroxystearat oder Polyglycerindimerat. Ebenfalls geeignet sind Gemische von Verbindungen aus mehreren dieser Substanzklassen;
(c7) Anlagerungsprodukte von 2 bis 15 Mol Ethylenoxid an Ricinusöl und/oder gehärtetes Ricinusöl;
(c8) Partialester auf Basis linearer, verzweigter, ungesättigter bzw. gesättigter C_{6/22}-Fettsäuren, Ricinolsäure sowie 12-Hydroxystearinsäure und Glycerin, Polyglycerin, Pentaerythrit, Dipentaerythrit, Zuckeralkohole (z.B. Sorbit), Alkylglucoside (z.B. Methylglucosid, Butylglucosid, Laurylglucosid) sowie Polyglucoside (z.B. Cellulose);
(c9) Mono-, Di- und Trialkylphosphate sowie Mono-, Di- und/oder Tri-PEG-alkylphosphate und deren Salze;
(c10) Wollwachsalkohole;
(c11) Polysiloxan-Polyalkyl-Polyether-Copolymere bzw. entsprechende Derivate;
(c12) Mischester aus Pentaerythrit, Fettsäuren, Citronensäure und Fettalkohol gemäß **DE-PS 1165574** und/oder Mischester von Fettsäuren mit 6 bis 22 Kohlenstoffatomen, Methylglucose und Polyolen, vorzugsweise Glycerin oder Polyglycerin sowie
(c13) Polyalkylenglycole
(c14) Betaine
(c15) Esterquats.

Die Anlagerungsprodukte von Ethylenoxid und/oder von Propylenoxid an Fettalkohole, Fettsäuren, Alkylphenole, Glycerinmono- und -diester sowie Sorbitanmono- und -diester von Fettsäuren oder an Ricinusöl stellen bekannte, im Handel erhältliche Produkte dar. Es handelt sich dabei um Homologengemische, deren mittlerer Alkoxylierungsgrad dem Verhältnis der Stoffmengen von Ethylenoxid und/oder Propylenoxid und Substrat, mit denen die Anlagerungsreaktion durchgeführt wird, entspricht. C_{12/18}-Fettsäuremono- und -diester von Anlagerungsprodukten von Ethylenoxid an Glycerin sind aus **DE-PS 20 24 051** als Rückfettungsmittel für kosmetische Zubereitungen bekannt.

C_{8/18}-Alkylmono- und -oligoglycoside, ihre Herstellung und ihre Verwendung sind aus dem Stand der Technik bekannt. Ihre Herstellung erfolgt insbesondere durch Umsetzung von Glucose oder Oligosacchariden mit primären Alkoholen mit 8 bis 18 C-Atomen. Bezüglich des Glycosidrestes gilt, daß sowohl Monoglycoside, bei denen ein cyclischer Zuckerrest glycosidisch an den Fettalkohol gebunden ist, als auch oligomere Glycoside mit einem Oligomerisationsgrad bis vorzugsweise etwa 8 geeignet sind. Der Oligomerisierungsgrad ist dabei ein statistischer Mittelwert, dem eine für solche technischen Produkte übliche Homologenverteilung zugrunde liegt.

Weiterhin können als Emulgatoren zwitterionische Tenside verwendet werden. Als zwitterionische Tenside werden solche oberflächenaktiven Verbindungen bezeichnet, die im Molekül mindestens eine quartäre Ammoniumgruppe und mindestens eine Carboxylat- und eine Sulfonatgruppe tragen. Besonders geeignete zwitterionische Tenside sind die sogenannten Betaine wie die N-Alkyl-N,N-dimethylammoniumglycinate, beispielsweise das Kokosalkyldimethylammoniumglycinat, N-Acylaminopropyl-N,N-dimethylammoniumglycinate, beispielsweise das Kokosacylaminopropyldimethylammoniumglycinat, und 2-Alkyl-3-carboxylmethyl-3-hydroxyethylimidazoline mit jeweils 8 bis 18 C-Atomen in der Alkyl- oder Acylgruppe sowie das Kokosacylaminoethylhydroxyethylcarboxymethylglycinat. Besonders bevorzugt ist das unter der CTFA-Bezeichnung *Cocamidopropyl Betaine* bekannte Fettsäureamid-Derivat. Ebenfalls geeignete Emulgatoren sind ampholytische Tenside. Unter ampholytischen Tensiden werden solche oberflächenaktiven Verbindungen verstanden, die außer einer C_{8/18}-Alkyl- oder -Acylgruppe im Molekül mindestens eine freie Aminogruppe und mindestens eine -COOH- oder -SO₃H-Gruppe enthalten und zur Ausbildung innerer Salze befähigt sind. Beispiele für geeignete ampholytische Tenside sind N-Alkylglycine, N-Alkylpropionsäuren, N-Alkylaminobuttersäuren, N-Alkyliminodipropionsäuren, N-Hydroxyethyl-N-alkylamidopropylglycine, N-Alkyltaurine, N-Alkylsarcosine, 2-Alkylaminopropionsäuren und Alkylaminoessigsäuren mit jeweils etwa 8 bis 18 C-Atomen in der Alkylgruppe. Besonders bevorzugte ampholytische Tenside sind das N-Kokosalkylaminopropionat, das Kokosacylaminoethylaminopropionat und das C_{12/18}-Acylsarcosin. Neben den ampholytischen kommen auch quartäre Emulgatoren in Betracht, wobei solche vom Typ der Esterquats, vorzugsweise methylquaternierte Difettsäuretriethanolaminester-Salze, besonders bevorzugt sind. Des weiteren können als anionische Emulgatoren Alkylethersulfate, Monoglyceridsulfate, Fettsäuresulfate, Sulfosuccinate und/oder Ethercarbonsäuren eingesetzt werden. Die Emulgatoren können in den erfindungsgemäßen Mitteln in Mengen von 0,1 bis 10, vorzugsweise 1 bis 5 Gew.-% - bezogen auf das Mittel - enthalten sein.

### Ölkörper

Als Ölkörper (Komponente d) kommen Guerbetalkohole auf Basis von Fettalkoholen mit 6 bis 18, vorzugsweise 8 bis 10 Kohlenstoffatomen, Ester von linearen C₆-C₂₂-Fettsäuren mit linearen C₆-C₂₂-Fettalkoholen, Ester von verzweigten C₆-C₁₃-Carbonsäuren mit linearen C₆-C₂₂-Fettalkoholen, Ester von linearen C₆-C₂₂-Fettsäuren mit verzweigten Alkoholen, insbesondere 2-Ethylhexanol, Ester von linearen und/oder verzweigten Fettsäuren mit mehrwertigen Alkoholen (wie z.B. Propylenglycol, Dimerdiol oder Trimertriol) und/oder Guerbetalkoholen, Triglyceride auf Basis C₆-C₁₀-Fettsäuren, flüssige Mono-/Di-/Triglyceridmischungen auf Basis von C₆-C₁₈-Fettsäuren, Ester von C₆-C₂₂-Fettalkoholen und/oder Guerbetalkoholen mit aromatischen Carbonsäuren, insbesondere Benzoesäure, Ester von C₂-C₁₂-Dicarbonsäuren mit linearen oder verzweigten Alkoholen mit 1 bis 22 Kohlenstoffatomen oder Polyolen mit 2 bis 10 Kohlenstoffatomen und 2 bis 6 Hydroxylgruppen, pflanzliche Öle, verzweigte primäre Alkohole, substituierte Cyclohexane, lineare C₆-C₂₂-Fettalkoholcarbonate, Guerbetcarbonate, Ester der Benzoesäure mit linearen und/oder verzweigten C₆-C₂₂-Alkoholen (z.B. Finsolv® TN), Dialkylether, Ringöffnungsprodukte von epoxidierten Fettsäureestern mit Polyolen, Siliconöle und/oder aliphatische bzw. naphthenische Kohlenwasserstoffe in Betracht. Als Ölkörper können ferner auch Siliconverbindungen eingesetzt werden, beispielsweise Dimethylpolysiloxane, Methylphenylpolysiloxane, cyclische Silicone sowie amino-, fettsäure-, alkohol-, polyether-, epoxy-, fluor-, alkyl- und/oder glykosidmodifizierte Siliconverbindungen, die bei Raumtemperatur sowohl flüssig als auch harzförmig vorliegen können. Die Ölkörper können in den erfindungsgemäßen Mitteln in Mengen von 1 bis 90, vorzugsweise 5 bis 80 und insbesondere 10 bis 50 Gew.-% - bezogen auf das Mittel - enthalten sein.

Ein weiterer Gegenstand der Erfindung betrifft die Verwendung von Desoxyribonucleinsäuren und UV-Lichtschutzfiltern zur Herstellung von Sonnenschutzmitteln und After-Sun Präparaten.

### Gewerbliche Anwendbarkeit

Die erfindungsgemäßen Mittel zeichnen sich durch eine hohe und gezielte Schutzwirkung und gleichzeitige Phasenstabilität bei besonders vorteilhafter hautkosmetischer Verträglichkeit aus. Dabei können die erfindungsgemäßen Mittel beispielsweise als Creme, Milch, Öl, Gele oder auch Lippenstift vorliegen. Typische Zubereitungen enthalten 0,001 bis 2,5, vorzugsweise 0,1 bis 0,5 Gew.-%- bezogen auf das Mittel- der Komponente (a) Desoxyribonucleinsäuren und 1 bis 20, vorzugsweise 5 bis 15 Gew.-% -bezogen auf das Mittel- Komponente (b) UV-Lichtschutzfilter.

In einer besonders bevorzugten Ausführungsform haben die Zubereitungen folgende Zusammensetzung
(a) 0,001 bis 2,5, vorzugsweise 0,1 bis 0,5 Gew.-% Desoxyribonucleinsäuren
(b) 1 bis 20, vorzugsweise 5 bis 15 Gew.-% UV-Lichtschutzfilter
(c) 0,1 bis 10, vorzugsweise 1 bis 5 Gew.-% Emulgatoren und
(d) 1 bis 90, vorzugsweise 5 bis 80 Gew.-% Ölkörper.

Die Angabe der Gew.-% bezieht sich dabei auf das fertige Mittel. Innerhalb der genannten Konzentrationsbereiche werden besonders stabile und feinteilige Emulsionen erhalten.

Als weitere Inhaltsstoffe können sie in untergeordneten Mengen weitere, mit den anderen Inhaltsstoffen kompatible Tenside enthalten. Typische Beispiele für **anionische Tenside** sind Seifen, Alkylbenzolsulfonate, Alkansulfonate, Olefinsulfonate, Alkylethersulfonate, Glycerinethersulfonate, α-Methylestersulfonate, Sulfofettsäuren, Alkylsulfate, Fettalkoholethersulfate, Glycerinethersulfate, Hydroxymischethersulfate, Monoglycerid(ether)sulfate, Fettsäureamid(ether)sulfate, Mono- und Dialkylsulfosuccinate, Mono- und Dialkylsulfosuccinamate, Sulfotriglyceride, Amidseifen, Ethercarbonsäuren und deren Salze, Fettsäureisethionate, Fettsäuresarcosinate, Fettsäuretauride, N-Acylaminosäuren, wie beispielsweise Acyllactylate, Acyltartrate, Acylglutamate und Acylaspartate, Alkyloligoglucosidsulfate, Proteinfettsäurekondensate (insbesondere pflanzliche Produkte auf Weizenbasis) und Alkyl(ether)phosphate. Sofern die anionischen Tenside Polyglycoletherketten enthalten, können diese eine konventionelle, vorzugsweise jedoch eine eingeengte Homologenverteilung aufweisen. Typische Beispiele für **nichtionische Tenside** sind Fettalkoholpolyglycolether, Alkylphenolpolyglycolether, Fettsäurepolyglycolester, Fettsäureamidpolyglycolether, Fettaminpolyglycolether, alkoxylierte Triglyceride, Mischether bzw. Mischformale, gegebenenfalls partiell oxidierte Alk(en)yloligoglykoside bzw. Glucoronsäurederivate, Fettsäure-N-alkylglucamide, Proteinhydrolysate (insbesondere pflanzliche Produkte auf Weizenbasis), Polyolfettsäureester, Zuckerester, Sorbitanester, Polysorbate und Aminoxide. Sofern die nichtionischen Tenside Polyglycoletherketten enthalten, können diese eine konventionelle, vorzugsweise jedoch eine eingeengte Homologenverteilung aufweisen. Typische Beispiele für **kationische Tenside** sind quartäre Ammoniumverbindungen und Esterquats, insbesondere quaternierte Fettsäuretrialkanolaminestersalze. Typische Beispiele für **amphotere bzw. zwitterionische Tenside** sind Alkylbetaine, Alkylamidobetaine, Aminopropionate, Aminoglycinate, Imidazoliniumbetaine und Sulfobetaine. Bei den genannten Tensiden handelt es sich ausschließlich um bekannte Verbindungen. Hinsichtlich Struktur und Herstellung dieser Stoffe sei auf einschlägige Übersichtsarbeiten beispielsweise **J.Falbe (ed.), "Surfactants in Consumer Products", Springer Verlag, Berlin, 1987, S. 54-124** oder **J.Falbe (ed.), "Katalysatoren, Tenside und Mineralöladditive", Thieme Verlag, Stuttgart, 1978, S. 123-217** verwiesen.

Die erfindungsgemäßen Mittel können ferner als weitere Hilfs- und Zusatzstoffe Co-Emulgatoren, Überfettungsmittel, Stabilisatoren, Wachse, Konsistenzgeber, Verdickungsmittel, Kationpolymere, biogene Wirkstoffe, Konservierungsmittel, Hydrotrope, Solubilisatoren, Farb- und Duftstoffe enthalten.

Als **Überfettungsmittel** können Substanzen wie beispielsweise Lanolin und Lecithin sowie polyethoxylierte oder acylierte Lanolin- und Lecithinderivate, Polyolfettsäureester, Monoglyceride und Fettsäurealkanolamide verwendet werden, wobei die letzteren gleichzeitig als Schaumstabilisatoren dienen.
Als **Perlglanzwachse** kommen beispielsweise in Frage: Alkylenglycolester, speziell Ethylenglycoldistearat; Fettsäurealkanolamide, speziell Kokosfettsäurediethanolamid; Partialglyceride, speziell Stearinsäuremonoglycerid; Ester von mehrwertigen, gegebenenfalls hydroxysubstituierte Carbonsäuren mit Fettalkoholen mit 6 bis 22 Kohlenstoffatomen, speziell langkettige Ester der Weinsäure; Fettstoffe, wie beispielsweise Fettalkohole, Fettketone, Fettaldehyde, Fettether und Fettcarbonate, die in Summe mindestens 24 Kohlenstoffatome aufweisen, speziell Lauron und Distearylether; Fettsäuren wie Stearinsäure, Hydroxystearinsäure oder Behensäure, Ringöffnungsprodukte von Olefinepoxiden mit 12 bis 22 Kohlenstoffatomen mit Fettalkoholen mit 12 bis 22 Kohlenstoffatomen und/oder Polyolen mit 2 bis 15 Kohlenstoffatomen und 2 bis 10 Hydroxylgruppen sowie deren Mischungen.

Als **Konsistenzgeber** kommen in erster Linie Fettalkohole mit 12 bis 22 und vorzugsweise 16 bis 18 Kohlenstoffatomen und daneben Partialglyceride in Betracht. Bevorzugt ist eine Kombination dieser Stoffe mit Alkyloligoglucosiden und/oder Fettsäure-N-methylglucamiden gleicher Kettenlänge und/oder Polyglycerinpoly-12-hydroxystearaten. Geeignete **Verdickungsmittel** sind beispielsweise Polysaccharide, insbesondere Xanthan-Gum, Guar-Guar, Agar-Agar, Alginate und Tylosen, Carboxymethylcellulose und Hydroxyethylcellulose, ferner höhermolekulare Polyethylenglycolmono- und -diester von Fettsäuren, Polyacrylate, (z.B. Carbopole® von Goodrich oder Synthalene® von Sigma), Polyacrylamide, Polyvinylalkohol und Polyvinylpyrrolidon, Tenside wie beispielsweise ethoxylierte Fettsäureglyceride, Ester von Fettsäuren mit Polyolen wie beispielsweise Pentaerythrit oder Trimethylolpropan, Fettalkoholethoxylate mit eingeengter Homologenverteilung oder Alkyloligoglucoside sowie Elektrolyte wie Kochsalz und Ammoniumchlorid.

Geeignete **kationische Polymere** sind beispielsweise kationische Cellulosederivate, wie z.B. eine quaternierte Hydroxyethylcellulose, die unter der Bezeichnung Polymer JR 400® von Amerchol erhältlich ist, kationische Stärke, Copolymere von Diallylammoniumsalzen und Acrylamiden, quaternierte Vinylpyrrolidon/Vinyl-imidazol-Polymere, wie z.B. Luviquat® (BASF), Kondensationsprodukte von Polyglycolen und Aminen, quaternierte Kollagenpolypeptide, wie beispielsweise Lauryldimonium hydroxypropyl hydrolyzed collagen (Lamequat®L/Grünau), quaternierte Weizenpolypeptide, Polyethylenimin, kationische Siliconpolymere, wie z.B. Amidomethicone, Copolymere der Adipinsäure und Dimethylaminohydroxypropyldiethylentriamin (Cartaretine®/Sandoz), Copolymere der Acrylsäure mit Dimethyldiallylammoniumchlorid (Merquat® 550/Chemviron), Polyaminopolyamide, wie z.B. beschrieben in der **FR 2252840 A** sowie deren vernetzte wasserlöslichen Polymere, kationische Chitinderivate wie beispielsweise quaterniertes Chitosan, gegebenenfalls mikrokristallin verteilt, Kondensationsprodukte aus Dihalogenalkylen, wie z.B. Dibrombutan mit Bisdialkylaminen, wie z.B. Bis-Dimethylamino-1,3-propan, kationischer Guar-Gum, wie z.B. Jaguar® CBS, Jaguar® C-17, Jaguar® C-16 der Firma Celanese, quaternierte Ammoniumsalz-Polymere, wie z.B. Mirapol® A-15, Mirapol® AD-1, Mirapol® AZ-1 der Firma Miranol.

Als **anionische, zwitterionische, amphotere und nichtionische Polymere** kommen beispielsweise Vinylacetat/Crotonsäure-Copolymere, Vinylpyrrolidon/Vinylacrylat-Copolymere, Vinylacetat/Butylmaleat/Isobomylacrylat-Copolymere, Methylvinylether/Maleinsäureanhydrid-Copolymere und deren Ester, unvernetzte und mit Polyolen vernetzte Polyacrylsäuren, Acrylamidopropyltrimethylammoniumchlorid/Acrylat-Copolymere, Octylacrylamid/Methylmethacrylat/tert.Butylaminoethylmethacrylat/2-Hydroxypropylmethacrylat-Copolymere, Polyvinylpyrrolidon, Vinylpyrrolidon/Vinylacetat-Copolymere, Vinylpyrrolidon/Dimethylaminoethylmethacrylat/Vinylcaprolactam-Terpolymere sowie gegebenenfalls derivatisierte Celluloseether und Silicone in Frage.

Typische Beispiele für **Fette** sind Glyceride, als **Wachse** kommen u.a. Bienenwachs, Carnaubawachs, Candelillawachs, Montanwachs, Paraffinwachs oder Mikrowachse gegebenenfalls in Kombination mit hydrophilen Wachsen, z.B. Cetylstearylalkohol oder Partialglyceriden in Frage. Als **Stabilisatoren** können Metallsalze von Fettsäuren, wie z.B. Magnesium-, Aluminium- und/oder Zinkstearat eingesetzt werden. Unter **biogenen Wirkstoffen** sind beispielsweise Tocopherol, Tocopherolacetat, Tocopherolpalmitat, Ascorbinsäure, Retinol, Bisabolol, Allantoin, Phytantriol, Panthenol, AHA-Säuren, Aminosäuren, Ceramide, Pseudoceramide, essentielle Öle, Pflanzenextrakte und Vitaminkomplexe zu verstehen. Als **Antischuppenmittel** können Climbazol, Octopirox und Zinkpyrethion eingesetzt werden. Gebräuchliche **Filmbildner** sind beispielsweise Chitosan, mikrokristallines Chito-san, quaterniertes Chitosan, Polyvinylpyrrolidon, Vinylpyrrolidon-Vinylacetat-Copolymerisate, Polymere der Acrylsäurereihe, quaternäre Cellulose-Derivate, Kollagen, Hyaluronsäure bzw. deren Salze und ähnliche Verbindungen. Als **Quelimittel** für wäßrige Phasen können Montmorillonite, Clay Mineralstoffe, Pemulen sowie alkylmodifizierte Carbopoltypen (Goodrich) dienen.

Zur Verbesserung des Fließverhaltens können femer **Hydrotrope,** wie beispielsweise Ethanol, isopropylalkohol, oder Polyole eingesetzt werden. Polyole, die hier in Betracht kommen, besitzen vorzugsweise 2 bis 15 Kohlenstoffatome und mindestens zwei Hydroxylgruppen. Typische Beispiele sind
- Glycerin;
- Alkylenglycole, wie beispielsweise Ethylenglycol, Diethylenglycol, Propylenglycol, Butylenglycol, Hexylenglycol sowie Polyethylenglycole mit einem durchschnittlichen Molekulargewicht von 100 bis 1.000 Dalton;
- technische Oligoglyceringemische mit einem Eigenkondensationsgrad von 1,5 bis 10 wie etwa technische Diglyceringemische mit einem Diglyceringehalt von 40 bis 50 Gew.-%;
- Methyolverbindungen, wie insbesondere Trimethylolethan, Trimethylolpropan, Trimethylolbutan, Pentaerythrit und Dipentaerythrit;
- Niedrigalkylglucoside, insbesondere solche mit 1 bis 8 Kohlenstoffen im Alkylrest, wie beispielsweise Methyl- und Butylglucosid;
- Zuckeralkohole mit 5 bis 12 Kohlenstoffatomen, wie beispielsweise Sorbit oder Mannit,
- Zucker mit 5 bis 12 Kohlenstoffatomen, wie beispielsweise Glucose oder Saccharose;
- Aminozucker, wie beispielsweise Glucamin.

Als **Konservierungsmittel** eignen sich beispielsweise Phenoxyethanol, Formaldehydlösung, Parabene, Pentandiol oder Sorbinsäure. Als **Inseksten-Repellentien** kommen N,N-Diethyl-m-touluamid, 1,2-Pentandiol oder Insect repellent 3535 in Frage, als **Selbstbräuner** eignet sich Dihydroxyaceton.

Als **Parfümöle** seien genannt Gemische aus natürlichen und synthetischen Riechstoffen. Natürliche Riechstoffe sind Extrakte von Blüten (Lilie, Lavendel, Rosen, Jasmin, Neroli, Ylang-Ylang), Stengeln und Blättern (Geranium, Patchouli, Petitgrain), Früchten (Anis, Koriander, Kümmel, Wacholder), Fruchtschalen (Bergamotte, Zitrone, Orangen), Wurzeln (Macis, Angelica, Sellerie, Kardamon, Costus, Iris, Calmus), Hölzern (Pinien-, Sandel-, Guajak-, Zedern-, Rosenholz), Kräutern und Gräsern (Estragon, Lemongras, Salbei, Thymian), Nadeln und Zweigen (Fichte, Tanne, Kiefer, Latschen), Harzen und Balsamen (Galbanum, Elemi, Benzoe, Myrrhe, Olibanum, Opoponax). Weiterhin kommen tierische Rohstoffe in Frage, wie beispielsweise Zibet und Castoreum. Typische synthetische Riechstoffverbindungen sind Produkte vom Typ der Ester, Ether, Aldehyde, Ketone, Alkohole und Kohlenwasserstoffe. Riechstoffverbindungen vom Typ der Ester sind z.B. Benzylacetat, Phenoxyethylisobutyrat, p-tert.-Butylcyclohexylacetat, Linalylacetat, Dimethylbenzylcarbinylacetat, Phenylethylacetat, Linalylbenzoat, Benzylformiat, Ethylmethyl-phenylglycinat, Allylcyclohexylpropionat, Styrallylpropionat und Benzylsalicylat. Zu den Ethern zählen beispielsweise Benzylethylether, zu den Aldehyden z.B. die linearen Alkanale mit 8 bis 18 Kohlenstoffatomen, Citral, Citronellal, Citronellyloxyacetaldehyd, Cyclamenaldehyd, Hydroxycitronellal, Lilial und Bourgeonal, zu den Ketonen z.B. die Jonone, α-isomethylionon und Methylcedrylketon, zu den Alkoholen Anethol, Citronellol, Eugenol, Isoeugenol, Geraniol, Linalool, Phenylethylalkohol und Terpineol, zu den Kohlenwasserstoffen gehören hauptsächlich die Terpene und Balsame. Bevorzugt werden jedoch Mischungen verschiedener Riechstoffe verwendet, die gemeinsam eine ansprechende Duftnote erzeugen. Auch ätherische Öle geringerer Flüchtigkeit, die meist als Aromakomponenten verwendet werden, eignen sich als Parfümöle, z.B. Salbeiöl, Kamillenöl, Nelkenöl, Melissenöl, Minzenöl, Zimtblätteröl, Lindenblütenöl, Wacholderbeerenöl, Vetiveröl, Olibanöl, Galbanumöl, Labolanumöl und Lavandinöl. Vorzugsweise werden Bergamotteöl, Dihydromyrcenol, Lilial, Lyral, Citronellol, Phenylethylalkohol, α-Hexylzimtaldehyd, Geraniol, Benzylaceton, Cyclamenaldehyd, Linalool, Boisambrene Forte, Ambroxan, Indol, Hedione, Sandelice, Citronenöl, Mandarinenöl, Orangenöl, Allylamylglycolat, Cyclovertal, Lavandinöl, Muskateller Salbeiöl, β-Damascone, Geraniumöl Bourbon, Cyclohexylsalicylat, Vertofix Coeur, Iso-E-Super, Fixolide NP, Evernyl, Iraldein gamma, Phenylessigsäure, Geranylacetat, Benzylacetat, Rosenoxid, Romilliat, Irotyl und Floramat allein oder in Mischungen, eingesetzt.

Als **Farbstoffe** können die für kosmetische Zwecke geeigneten und zugelassenen Substanzen verwendet werden, wie sie beispielsweise in der Publikation **"Kosmetische Färbemittel" der Farbstoffkommission der Deutschen Forschungsgemeinschaft, Verlag Chemie, Weinheim, 1984, S.81-106** zusammengestellt sind. Diese Farbstoffe werden üblicherweise in Konzentrationen von 0,001 bis 0,1 Gew.-%, bezogen auf die gesamte Mischung, eingesetzt.

Der Gesamtanteil der Hilfs- und Zusatzstoffe kann 1 bis 50, vorzugsweise 5 bis 40 Gew.-% - bezogen auf die Mittel - betragen. Ein weiterer Gegenstand der Erfindung betrifft schließlich die Verwendung von Mitteln, enthaltend
(a) Desoxyribonucleinsäuren und
(b) UV-Lichtschutzfilter
zur Herstellung von Sonnenschutzmitteln sowie zur Herstellung von After-Sun Präparaten. Diese Mittel können in üblichen Formulierungen, wie Cremes, Lotionen, Milch, Ölen, aber auch Gelen und Lippenstiften vorliegen.

### Beispiele

Folgende Produkte wurde hergestellt und auf ihre Hautverträglichkeit getestet. Die Hautverträglichkeit wurde von 10 Probanden getestet. Verglichen wurden jeweils die erfindungsgemäßen Rezepturen im Vergleich zu den entsprechenden Basisrezepturen (ohne Lichtschutzfilter und marine DNA). "0" bedeutet kein Unterschied im Hautgefühl und in der Hautverträglichkeit "+" bedeutet spürbare Verbesserung, "++" bedeutet deutliche Verbesserung.

**Tab. 1**

| **Sonnenschutzmittel (Mengenangaben als Gew.-%)** | |
|---|---|
| **1a)** Sonnenschutzcreme | |
| Glyceryl Stearate (and) Ceteareth-12/20 (and) Cetearyl Alcohol (and) Cetyl Palmitate | 5,0 |
| Decyl Oleate | 3,0 |
| Cetearyl Isononanoate | 3,0 |
| Glycerin (86 Gew.-%ig) | 3,0 |
| Tocopherol | 3,0 |
| Desoxyribonucleinsäure ¹⁾ | 1,0 |
| Wasser | Ad 100 |
| | |

| **1b)** Sonnenschutzemulsion | |
|---|---|
| Glyceryl Stearate (and) Ceteareth-12/20 (and) Cetearyl alcohol (and) Cetyl Palmitate | 5,0 |
| Decyl Oleate | 3,0 |
| Cetearyl Isononanoate | 3,0 |
| Octyl Methoxycinnamate | 7,5 |
| Glycerin (86 Gew.-%ig) | 3,0 |
| Desoxyribonucleinsäure¹⁾ | 1,0 |
| Wasser | Ad 100 |

| | |
|---|---|
| ¹⁾ Desoxyribonucleinsäure: Molekuargewicht ca. 70000, Reinheit (bestimmt durch spektrophotometrische Messung der Absorption bei 260 nm sowie 280 nm): mindestens 1,7. | |

Sowohl Produkt 1a) als auch 1b) wurden hinsichtlich ihrer Hautverträglichkeit mit "++" bewertet.

**Tab. 2**

| **O/W-Sonnenschutzemulsionen (Mengenangaben als Gew.-%) Rezepturen 1 und 2** | | |
|---|---|---|
| Komponente | **1** | **2** |
| Octyl Dodecanol | 18,0 | 16,0 |
| Cetearyl Glucoside (and) Cetearyl Alcohol (50 : 50) | 4,5 | 4,2 |
| Desoxyribonucleinsäure (DNA)¹⁾ | 0,5 | 0,5 |
| Benzophenone-3 | - | 2,0 |
| Octyl Methoxycinnamate | - | 7,5 |
| Titandioxid | 5,0 | - |
| Zinkoxid | 5,0 | - |
| Octyl Triazone | - | 6,0 |
| Glycerin | 5,0 | 5,0 |
| Wasser | | |
| **Hautverträglichkeit** | **+** | **+** |

| | | |
|---|---|---|
| ¹⁾ Desoxyribonucleinsäure: Molekuargewicht ca. 70000, Reinheit (bestimmt durch spektrophotometrische Messung der Absorption bei 260 nm sowie 280 nm): mindestens 1,7. | | |

**Tab. 3**

| **Sonnenschutzlotion (Mengenangaben als Gew.-%) Rezepturen 3 bis 7** | | | | | | |
|---|---|---|---|---|---|---|
| Komponente | Handelsname | **3** | **4** | **5** | **6** | **7** |
| Glyceryl stearate, Ceteareth-20, Ceteareth-12, Cetearyl Alcohol, Cetyl palmitate | Emulgade SE | 6,0 | 4,7 | 8,0 | 6,0 | 8,0 |
| C16-C18 Fettalkohol | Lanette 0 | 1,5 | - | 1,0 | - | 1,0 |
| Decyloleat | Cetiol V | 4,0 | - | - | - | - |
| Octylstearat | Cetiol 868 | 8,0 | 6,0 | - | - | - |
| Ceteareth-20 | Eumulgin-B-2 | | 1,3 | - | - | - |
| marine DNA¹⁾ | Hydagen MDNA¹⁾ | 0,5 | 0,5 | 0,5 | 0,5 | 0,5 |
| Coco-caprylate/caprate | Cetiol LC | | | | 3,0 | 3,0 |
| Glycerol | Glycerin (86 gew.-%ig) | 5,0 | 5,0 | 3,0 | 3,0 | 3,0 |
| Shea Butter | Cetiol SB 45 | | | | 2,0 | 2,0 |
| Cetearyl isononanoate | Cetiol SN | - | 6,0 | 3,0 | 3,0 | 3,0 |
| Isoamyl-p-methoxycinnamate | Neo-Heliopan E 1000 | 4,0 | 4,0 | 4,0 | - | - |
| Benzophenone-3 | Neo-Heliopan BB (=Uvinul M 40) | 1,0 | 1,0 | 1,0 | 1,0 | 1,0 |
| Octyl-methoxycinnamate | Parsol MCX | - | - | - | 7,5 | 7,5 |
| Butyl methoxydibenzoylmethane | Parsol 1789 | - | - | - | 2,5 | 2,5 |
| Carbomer | Carbopol-980 | - | - | - | 15,0 | 15,0 |
| (Polyacrylsäure) | (2 gew.-%ig) | | | | | |
| Natriumhydroxidlösung | Natronlauge (20 gew.-%ig) | - | - | - | 0,18 | 0,18 |
| Tocopherol | Copherol F 1300 | 1,0 | 1,0 | 1,0 | - | - |
| Tocopherolacetat | Copherol 1250 | - | - | - | 1,0 | 1,0 |
| Konservierungsstoffe | Phenonip | 0,2 | 0,2 | 0,2 | 0,2 | 0,2 |
| Wasser | | | | | | |
| | | | | | | |
| **Hautverträglichkeit** | | **++** | **++** | **++** | **++** | **++** |

| | | | | | | |
|---|---|---|---|---|---|---|
| ¹⁾ Desoxyribonucleinsäure: Molekuargewicht ca. 70000, Reinheit (bestimmt durch spektrophotometrische Messung der Absorption bei 260 nm sowie 280 nm): mindestens 1,7. | | | | | | |

**Tab. 4**

| **Sonnenschutzlotion (Mengenangaben als Gew.-%) Rezepturen 8 und 9** | | | |
|---|---|---|---|
| Komponente | Handelsname | **8** | **9** |
| Glyceryl stearate, Cetearyl stearate, | Cutina CBS | 8,0 | - |
| Cetyl palmitate, Coco-glycerides Glyceryl stearate, Ceteareth-20, | Emulgade SE | - | 6,0 |
| Ceteareth-12, Cetearyl Alcohol, Cetyl palmitate | | | |
| Ceteareth-12 | Eumulgin-B-1 | 4,0 | - |
| C 16-C 18 Fettalkohol | Lanette 0 | 2,0 | - |
| Dioctylcyclohexane | Cetiol S | 7,0 | 6,0 |
| Octyldodecanol | Eutanol G | 4,5 | 4,0 |
| Shea butter | Cetiol SB 45 | 2,0 | 2,0 |
| Tocopherol | Copherol F 1300 | 3,0 | 3,0 |
| Titandioxide | Hombitec-L-5 | 2,0 | - |
| Octyl-methoxycinnamate | Parsol MCX | 7,5 | 7,5 |
| Butyl methoxydibenzoylmethane | Parsol 1789 | 2,0 | 2,0 |
| Trennmittel | Baysilon M 350 | 1,0 | 1,0 |
| Glyceryl stearate | Cutina MD | - | 2,0 |
| Zinkoxid | Zinkoxid | - | 2,0 |
| Glycerol | Glycerin (86 gew.-%ig) | 3,0 | 3,0 |
| Marine Desoxyribonucleinsäure ¹⁾ | Hydagen MDNA¹⁾ | 0,5 | 0,5 |
| Magnesium aluminium silicate | Veegum | - | 1,0 |
| Xanthan gum | Keitrol BT | - | 0,5 |
| Wasser | | ad 100 | |
| | | | |
| **Hautverträglichkeit** | | **++** | **+** |

| | | | |
|---|---|---|---|
| ¹⁾ Desoxyribonucleinsäure: Molekuargewicht ca. 70000, Reinheit (bestimmt durch spektrophotometrische Messung der Absorption bei 260 nm sowie 280 nm): mindestens 1,7. | | | |

**Tab. 5**

| **Lippenschutzstifte (Mengenangaben als Gew.-%) Rezepturen 10 und 11** | | | |
|---|---|---|---|
| Komponente | Handelsname | 10 | 11 |
| Cera alba bees wax | Bienenwachs 8100 | 6,5 | 5,0 |
| Candelilla wax | Candelilla Wax | 7,0 | 5,0 |
| Camauba wax | Camauba Wax | 5,5 | 7,0 |
| Ricinusfetsäuretiglycerid | Castor Oel | 18,0 | 18,0 |
| Tocopherol | Copherol F 1300 | 2,0 | 2,0 |
| Polyglyceryl-2-Dipolyhydroxystearate | Dehymuls PGPH | 4,0 | 4,0 |
| Octyldodecanol | Eutanol G | 17,0 | 17,0 |
| | Farbpigmente | 2,0 | |
| Soybean sterol | Generol 122 N | 2,5 | |
| Glyceryl laurate | Monomuls 90-L 12 | 3,0 | 3,0 |
| Caprylic/capric triglyceride | Myritol 318 | 14,0 | 14,0 |
| Propylene glycol dicaprylate/dicaprate | Myritol PC | 6,0 | 6,0 |
| Marine DNA¹⁾ | Hydagen MDNA¹⁾ | 0,5 | 0,5 |
| Butyl methoxydibenzoylmethane | Parsol 1789 | 2,5 | 2,5 |
| Benzophenone-3 | Neo-Heliopan BB | 1,0 | 1,0 |
| Aqua | Wasser | Ad 100 | |
| | | | |
| **Hautverträglichkeit** | | **++** | **++** |

| | | | |
|---|---|---|---|
| ¹⁾ Desoxyribonucleinsäure: Molekuargewicht ca. 70000, Reinheit (bestimmt durch spektrophotometrische Messung der Absorption bei 260 nm sowie 280 nm): mindestens 1,7. | | | |

## Patentansprüche

1. Kosmetische Mittel, enthaltend
(a) 0.001 bis 0.5 Gew. % (Desoxy)ribonucleinsäuren und
(b) 1 bis 20 Gew. % UV-Lichtschutzfilter

2. Kosmetische Mittel nach Anspruch 1, **dadurch gekennzeichnet, daß** sie als weitere Komponenten
(c) Emulgatoren und (d) Ölkörper enthalten.

3. Mittel nach den Ansprüchen 1 und 2, **dadurch gekennzeichnet, daß** sie UV-Lichtschutzfilter enthalten, die ausgewählt sind aus der Gruppe, die gebildet wird von 3-Benzylidencampher und dessen Derivaten, 4-Aminobenzoesäurederivaten, Estern der Zimtsäure, Estern der Salicylsäure, Derivaten des Benzophenons, Estern der Benzalmalonsäure, Triazinderivaten, Propan-1,3-dionen, 2-Phenylbenzimidazol-5-sulfonsäure und deren Salzen, Sulfonsäurederivaten von Benzophenonen, Sulfonsäurederivaten des 3-Benzylidencamphers, sowie Benzoylmethanderivaten.

4. Mittel nach den Ansprüchen 1 bis 3, **dadurch gekennzeichnet, daß** sie als UV-Lichtschutzfilter feindisperse Metalloxide bzw. Salze enthalten, die ausgewählt sind aus der Gruppe, die gebildet wird von Titandioxid, Zinkoxid, Eisenoxid, Aluminiumoxid, Ceroxid, Zirkoniumoxid, Silicate (Talk), Bariumsulfat und Zinkstearat.

5. Mittel nach den Ansprüchen 1 bis 4, **dadurch gekennzeichnet, daß** sie als UV-Lichtschutzmittel Antioxidantien enthalten, die ausgewählt sind aus der Gruppe, die gebildet wird von Superoxid-Dismutase, Tocopherolen (Vitamin E) und Ascorbinsäure (Vitamin C).

6. Mittel nach Anspruch 1, **dadurch gekennzeichnet, daß** sie Ölkörper enthalten, die ausgewählt sind aus der Gruppe, die gebildet wird von Guerbetalkoholen auf Basis von Fettalkoholen mit 6 bis 18, Estern von linearen C₆-C₂₂-Fettsäuren mit linearen C₆-C₂₂-Fettalkoholen, Estern von verzweigten C₆-C₁₃-Carbonsäuren mit linearen C₆-C₂₂-Fettalkoholen, Estern von linearen C₆-C₂₂-Fettsäuren mit verzweigten Alkoholen, Estern von linearen und/oder verzweigten Fettsäuren mit mehrwertigen Alkoholen und/oder Guerbetalkoholen, Triglyceriden auf Basis C₆-C₁₀-Fettsäuren, flüssigen Mono-/Di-/Triglyceridmischungen auf Basis von C₆-C₁₈-Fettsäuren, Estern von C₆-C₂₂-Fettalkoholen und/oder Guerbetalkoholen mit aromatischen Carbonsäuren, Estern von C₂-C₁₂-Dicarbonsäuren mit linearen oder verzweigten Alkoholen mit 1 bis 22 Kohlenstoffatomen oder Polyolen mit 2 bis 10 Kohlenstoffatomen und 2 bis 6 Hydroxylgruppen, pflanzliche Ölen, verzweigten primären Alkoholen, substituierten Cyclohexanen, linearen C₆-C₂₂-Fettalkoholcarbonaten, Guerbetcarbonaten, Estern der Benzoesäure mit linearen und/oder verzweigten C₆-C₂₂-Alkoholen, Dialkylethern, Ringöffnungsprodukten von epoxidierten Fettsäureestern mit Polyolen, aliphatischen bzw. naphthenischen Kohlenwasserstoffen sowie Siliconölen.

7. Mittel nach den Ansprüchen 1 bis 6, **dadurch gekennzeichnet, daß** sie Emulgatoren enthalten, die ausgewählt sind aus der Gruppe, die gebildet wird von
(c1) Anlagerungsprodukten von 2 bis 30 Mol Ethylenoxid undl oder 0 bis 5 Mol Propylenoxid an lineare Fettalkohole mit 8 bis 22 C-Atomen, an Fettsäuren mit 12 bis 22 C-Atomen und an Alkylphenole mit 8 bis 15 C-Atomen in der Alkylgruppe;
(c2) C_{12/18}-Fettsäuremono- und -diestern von Anlagerungsprodukten von 1 bis 30 Mol Ethylenoxid an Glycerin;
(c3) Glycerinmono- und -diestern und Sorbitanmono- und -diestern von gesättigten und ungesättigten Fettsäuren mit 6 bis 22 Kohlenstoffatomen und deren Ethylenoxidanlagerungsprodukten;
(c4) Alkylmono- und -oligoglycosiden mit 8 bis 22 Kohlenstoffatomen im Alkylrest und deren ethoxylierten Analoga;
(c5) Anlagerungsprodukten von 15 bis 60 Mol Ethylenoxid an Ricinusöl und/oder gehärtetes Ricinusöl;
(c6) Polyol- und insbesondere Polyglycerinestern;
(c7) Anlagerungsprodukten von 2 bis 15 Mol Ethylenoxid an Ricinusöl und/oder gehärtetes Ricinusöl;
(c8) Partialestern auf Basis linearer, verzweigter, ungesättigter bzw. gesättigter C_{6/22}-Fettsäuren, Ricinolsäure sowie 12-Hydroxystearinsäure und Glycerin, Polyglycerin, Pentaerythrit, Dipentaerythrit, Zuckeralkoholen, Alkylglucosiden sowie Polyglucosiden;
(c9) Mono-, Di- und Trialkylphosphaten sowie Mono-, Di- undloder Tri-PEG-alkylphosphaten;
(c10) Wollwachsalkoholen;
(c11) Polysiloxan-Polyalkyl-Polyether-Copolymeren bzw. entsprechende Derivaten;
(c12) Mischestern aus Pentaerythrit, Fettsäuren, Citronensäure und Fettalkohol und/oder Mischester von Fettsäuren mit 6 bis 22 Kohlenstoffatomen, Methylglucose und Polyolen;
(c13) Polyalkylenglycolen;
(c14) Betainen;
(c15) Esterquats.

8. Verwendung von Mitteln, enthaltend
(a) 0.001 bis 0.5 Gew. % (Desoxy)ribonucleinsäuren und
(b) 1 bis 20 Gew. % UV-Lichtschutzfilter
zur Herstellung von Sonnenschutzmitteln.

9. Verwendung von Mitteln, enthaltend
(a) 0.001 bis 0.5 Gew. % (Desoxy)ribonucleinsäuren und
(b) 1 bis 20 Gew. % UV-Lichtschutzfilter
zur Herstellung von After-Sun Präparaten.

## Claims

1. Cosmetic compositions containing
a) 0.001 to 0.5% by weight (deoxy)ribonucleic acids and
b) 1 to 20% by weight UV filters.

2. Cosmetic compositions as claimed in claim 1, **characterized in that** they contain emulsifiers c) and oil components d) as further components.

3. Compositions as claimed in claims 1 and 2, **characterized in that** they contain UV filters selected from the group consisting of 3-benzylidene camhor and derivatives thereof, 4-aminobenzoic acid derivatives, esters of cinnamic acid, esters of salicylic acid, derivatives of benzophenone, esters of benzal malonic acid, triazine derivatives, propane-1,3-diones, 2-phenylbenzimidazole-5-sulfonic acid and salts thereof, sulfonic acid derivatives of benzophenones, sulfonic acid derivatives of 3-benzylidene camphor and benzoyl methane derivatives.

4. Compositions as claimed in claims 1 to 3, **characterized in that** they contain finely dispersed metal oxides or salts selected from the group consisting of titanium dioxide, zinc oxide, iron oxide, aluminium oxide, cerium oxide, zirconium oxide, silicate (talcum), barium sulfate and zinc stearate as UV filters.

5. Compositions as claimed in claims 1 to 4, **characterized in that** they contain antioxidants selected from the group consisting of Superoxid-Dismutase, tocopherols (vitamin E) and ascorbic acid (vitamin C) as UV filters.

6. Compositions as claimed in claim 1, **characterized in that** they contain oil components selected from the group consisting of Guerbet alcohols based on fatty alcohols containing 6 to 18 carbon atoms, esters of linear C₆₋₂₂ fatty acids with linear C₆₋₂₂ fatty alcohols, esters of branched C₆₋₁₃ carboxylic acids with linear C₆₋₂₂ fatty alcohols, esters of linear C₆₋₂₂ fatty acids with branched alcohols, esters of linear and/or branched fatty acids with polyhydric alcohols and/or Guerbet alcohols, triglycerides based on C₆₋₁₀ fatty acids, liquid mono-/di-/triglyceride mixtures based on C₆₋₁₈ fatty acids, esters of C₆₋₂₂ fatty alcohols and/or Guerbet alcohols with aromatic carboxylic acids, esters of C₂₋₁₂ dicarboxylic acids with linear or branched alcohols containing 1 to 22 carbon atoms or polyols containing 2 to 10 carbon atoms and 2 to 6 hydroxyl groups, vegetable oils, branched primary alcohols, substituted cyclohexanes, linear C₆₋₂₂ fatty alcohol carbonates, Guerbet carbonates, esters of benzoic acid with linear and/or branched C₆₋₂₂ alcohols, dialkyl ethers, ring opening products of epoxidized fatty acid esters with polyols, aliphatic or naphthenic hydrocarbons and silicone oils.

7. Compositions as claimed in claims 1 to 6, **characterized in that** they contain emulsifiers selected from the group consisting of
(c1) products of the addition of 2 to 30 moles of ethylene oxide and/or 0 to 5 moles of propylene oxide onto linear fatty alcohols containing 8 to 22 carbon atoms, onto fatty acids containing 12 to 22 carbon atoms and onto alkylphenols containing 8 to 15 carbon atoms in the alkyl group;
(c2) C_{12/18} fatty acid monoesters and diesters of products of the addition of 1 to 30 moles of ethylene oxide onto glycerol;
(c3) glycerol monoesters and diesters and sorbitan monoesters and diesters of saturated and unsaturated fatty acids containing 6 to 22 carbon atoms and ethylene oxide adducts thereof;
(c4) alkyl mono- and oligoglycosides containing 8 to 22 carbon atoms in the alkyl group and ethoxylated analogs thereof;
(c5) adducts of 15 to 60 moles of ethylene oxide with castor oil and/or hydrogenated castor oil;
(c6) polyol esters and, in particular, polyglycerol esters;
(c7) products of the addition of 2 to 15 moles of ethylene oxide onto castor oil and/or hydrogenated castor oil;
(c8) partial esters based on linear, branched, unsaturated or saturated C_{6/22} fatty acids, ricinoleic acid and 12-hydroxystearic acid and glycerol, polyglycerol, pentaerythritol, dipentaerythritol, sugar alcohols, alkyl glucosides and polyglucosides;
(c9) mono-, di- and trialkyl phosphates and mono-, di- and/or tri-PEG-alkyl phosphates;
(c10) wool wax alcohols;
(c11) polysiloxane/polyalkyl polyether copolymers and corresponding derivatives;
(c12) mixed esters of pentaerythritol, fatty acids, citric acid and fatty alcohol and/or mixed esters of fatty acids containing 6 to 22 carbon atoms, methyl glucose and polyols;
(c13) polyalkylene glycols;
(c14) betaines;
(c15) esterquats.

8. The use of compositions containing
(a) 0.001 to 0.5% by weight (desoxy)ribonucleic acids and
(b) 1 to 20% by weight UV filters
for the production of sun protection compositions.

9. The use of compositions containing
(a) 0.001 to 0.5% by weight (desoxy)ribonucleic acids and
(b) 1 to 20% by weight UV filters
for the production of after-sun preparations.

## Revendications

1. Préparations cosmétiques contenant :
a) de 0,001 à 0,5 % en poids d'acides (désoxy)ribonucléiques et,
b) de 1 à 20 % en poids de filtre protecteur contre la lumière UV.

2. Préparations cosmétiques selon la revendication 1,
**caractérisées en ce qu'**
elles renferment comme autres composants :
c) des agents émulsionnants, et
d) des composés huileux.

3. Préparations cosmétiques selon l'une quelconque des revendications 1 et 2,
**caractérisées en ce qu'**
elles contiennent des filtres protecteurs contre la lumière UV qui sont choisis dans le groupe qui est formé du 3-benzylidène camphre, et de ses dérivés, de dérivés de l'acide 4-aminobenzoïque, d'esters d'acide cinnamique, d'esters de l'acide salicylique, de dérivés de la benzophénone, d'esters de l'acide benzalmalonique, de dérivés de la triazine, de propane-1,3-diones, de l'acide 2-phénylbenzimidazolyl-5-sulfonique, et de ses sels, de dérivés d'acide sulfonique de benzophénones, de dérivés acide sulfonique du 3-benzylidène camphre ainsi que de dérivés du benzoylméthane.

4. Préparations selon l'une quelconque des revendications 1 à 3,
**caractérisées en ce qu'**
elles contiennent comme filtre protecteur contre la lumière UV, des oxydes métalliques finement dispersés ou des sels qui sont choisis dans le groupe qui est formé du dioxyde de titane, de l'oxyde de zinc, de l'oxyde de fer, de l'oxyde d'aluminium, de l'oxyde de cérium, de l'oxyde de zirconium, de silicates (talc), du sulfate de baryum et du stéarate de zinc.

5. Préparations selon l'une quelconque des revendications 1 à 4,
**caractérisées en ce qu'**
elles contiennent des antioxydants comme agent protecteur contre la lumière UV qui sont choisis dans le groupe formé de la superoxydismutase, des tocophérols (vitamine E) et de l'acide ascorbique (vitamine C).

6. Préparations selon la revendication 1,
**caractérisées en ce qu'**
elles contiennent des composés huileux qui sont choisis dans le groupe formé des alcools de Guerbet à base d'alcools gras ayant de 6 à 18 atomes de carbone, des esters d'acides gras linéaires en C₆-C₂₂ avec des alcools gras linéaires en C₆-C₂₂, des esters d'acides carboxyliques ramifiés en C₆-C₁₃ avec des alcools gras linéaires en C₆-C₂₂, des esters d'acides gras linéaires en C₆-C₂₂ avec des alcools ramifiés, des esters d'acides gras linéaires et/ou ramifiés avec des alcools plurifonctionnels et/ou des alcools de Guerbet, des triglycérides à base d'acides gras en C₆-C₁₀, des mélanges liquide de mono/di/triglycérides à base d'acides gras en C₆-C₁₈, des esters d'alcools gras en C₆-C₂₂ et/ou d'alcools de Guerbet avec des acides carboxyliques aromatiques, des esters d'acides dicarboxyliques en C₂-C₁₂ avec des alcools linéaires ou ramifiés ayant de 1 à 22 atomes de carbone ou des polyols ayants de 2 à 10 atomes de carbone et de 2 à 6 groupes hydroxyle, des huiles végétales, des alcools primaires ramifiés, des cyclohexanes substitués, des carbonates d'alcool gras linéaires en C₆-C₂₂, des esters d'acide benzoïque avec des alcools linéaires et/ou ramifiés en C₆-C₂₂, des éthers de dialkyle, des produits d'ouverture du cycle d'esters d'acide gras époxydés avec des polyols, des hydrocarbures aliphatiques ou naphténiques ainsi que des huiles de silicone.

7. Préparations selon l'une quelconque des revendications 1 à 6,
**caractérisées en ce qu'**
elles renferment des agents émulsionnants qui sont choisis dans le groupe formé :
c1) des produits d'addition de 2 à 30 mol d'oxyde d'éthylène et/ou de 0 à 5 mol d'oxyde de propylène, sur des alcools gras linéaires ayant de 8 à 22 atomes de carbone, sur des acides gras ayant de 12 à 22 atomes de carbone et sur des alkylphénols ayant de 8 à 15 atomes de carbone dans le groupe alkyle ;
c2) des mono- et des diesters d'acide gras en C₁₂-C₁₈ de produits d'addition de 1 à 30 mol d'oxyde d'éthylène sur le glycérol,
c3) des mono- et des diesters de glycérol et de mono- et de diesters de sorbitanne avec des acides gras saturés et non saturés, ayant de 6 à 22 atomes de carbone et leurs produits d'addition d'oxyde d'éthylène,
c4) des alkylmono- et oligoglycosides ayant de 8 à 22 atomes de carbone dans le reste alkyle et leurs analogues éthoxylés,
c5) des produits d'addition de 15 à 60 mol d'oxyde d'éthylène sur de l'huile de ricin et/ou de l'huile de ricin durcie,
c6) des esters de polyol et en particulier de polyglycérol,
c7) des produits d'addition de 2 à 15 mol d'oxyde d'éthylène sur l'huile de ricin et/ou l'huile de ricin durcie,
c8) des esters partiels à base d'acide gras linéaires, ramifiés, non saturés ou saturés, ayant de C₆ à C₂₂ atomes de carbone, l'acide ricinoléique ainsi que l'acide 12-hydroxystéarique et du glycérol, du polyglycérol, du péntaérythritol, du dipentaerythritol, des alcools de sucre, des alkylglucosides ainsi que des polyglucosides,
c9) des phosphates de mono-, di- et de trialkyle ainsi que des phosphates de mono-, de di- et de tri- PEG-alkyle et leurs sels,
c10) des alcools de cire de laine,
c11) des copolymères polysiloxane-polyalkyl-polyéther ou les dérivés correspondants,
c12) des esters mixtes à base de pentaerythritol, d'acide gras, d'acide citrique et d'alcool gras et/ou des esters mixtes d'acide gras d'acides gras ayant de 6 à 22 atomes de carbone du méthyl glucose et des polyols,
c13) des polyalkylène glycols,
c14) des bétaïnes,
c15) des esterquats.

8. Utilisation de préparations contenant :
a) de 0,001 à 0,5 % en poids d'acides (desoxy)ribonucléiques, et
b) de 1 à 20 % en poids de filtres protecteurs contre la lumière UV,
en vue de la production d'agents de protection solaire.

9. Utilisation de préparations contenant :
a) de 0,001 à 0,5 % en poids d'acides (desoxy)ribonucléiques, et
b) de 1 à 20 % en poids de filtres protecteurs contre la lumière UV,
en vue de la production d'agents de protection après solaire (after sun).
